# EUROPEAN PATENT SPECIFICATION

(11) **EP 2 887 068 B1**
(45) Date of publication and mention of the grant of the patent: **15.08.2018**
(21) Application number: 13306815.5
(22) Date of filing: 20.12.2013
(51) Int. Cl.: G01N 33/569

(54) **Method for improving the diagnosis of invasive Candida infections by FBA1 protein detection**
Verfahren zur Verbesserung der Diagnose von invasiven Candida-Infektionen durch FBA1-Proteinerkennung
Procédé pour améliorer le diagnostic d'infections invasives par Candida via la détection d'une protéine FBA1

(43) Date of publication of application: 24.06.2015
(73) Proprietor: Bio-Rad Innovations, 92430 Marnes-La-Coquette (FR); Centre Hospitalier Universitaire de Lille, 59000 Lille (FR); Université de Lille 2 Droit et Santé, 59000 Lille (FR)
(72) Inventor: Damiens, Sébastien, 59470 Merckeghem (FR); Tabouret, Marc Charles Victor, 59320 Ennetieres en Weppes (FR); Sendid, Boualem, 59120 Loos (FR); Poulain, Daniel, 59242 Templeuve (FR); Fradin Chantal, 59000 Lille (FR)
(74) Representative: Lavoix

(56) References cited:
- AIDA PITARCH ET AL: "Proteomics-based identification of novelCandida albicans antigens for diagnosis of systemic candidiasis in patients with underlying hematological malignancies", PROTEOMICS, vol. 4, no. 10, 1 October 2004 (2004-10-01), pages 3084-3106, XP055036868, ISSN: 1615-9853, DOI: 10.1002/pmic.200400903
- PONTON JOSE ET AL: "Advances and limitations in the early diagnosis of invasive yeasts infections", REVISTA IBEROAMERICANA DE MICOLOGIA, vol. 24, no. 3, September 2007 (2007-09), pages 181-186, XP002682704, ISSN: 1130-1406
- ANANE ET AL: "Diagnostic biologique des candidoses systemiques : difficultes et perspectives", PATHOLOGIE ET BIOLOGIE, L'EXPANSION SCIENTIFIQUE FRANCAISE, PARIS, FR, vol. 55, no. 5, 1 June 2007 (2007-06-01), pages 262-272, XP022113786, ISSN: 0369-8114, DOI: 10.1016/J.PATBIO.2006.03.003
- FANG-QIU LI ET AL: "Diagnostic value of immunoglobulin G antibodies against Candida enolase and fructose-bisphosphate aldolase for candidemia", BMC INFECTIOUS DISEASES, BIOMED CENTRAL, LONDON, GB, vol. 13, no. 1, 31 May 2013 (2013-05-31), page 253, XP021152048, ISSN: 1471-2334, DOI: 10.1186/1471-2334-13-253
- PITARCH AIDA ET AL: "Prediction of the clinical outcome in invasive candidiasis patients based on molecular fingerprints of five anti-Candida antibodies in serum.", MOLECULAR & CELLULAR PROTEOMICS : MCP JAN 2011, vol. 10, no. 1, January 2011 (2011-01), XP002725138, ISSN: 1535-9484

### SEQUENCE LISTING

<110> Bio-Rad Innovations
<120> METHOD FOR IMPROVING THE DIAGNOSIS OF INVASIVE CANDIDA INFECTIONS
   BY FBA1 PROTEIN DETECTION
<130> BET13P2406
<160> 4
<170> PatentIn version 3.5
<210> 1
   <211> 1080
   <212> DNA
   <213> Candida albicans
<220>
   <221> misc_feature
   <222> (1)..(1080)
   <223> Fructose bisphosphate aldolase
<400> 1
   atggctcctc cagcagtttt aagtaaatcc ggtgttatct acggtaaaga cgtcaaagac      60
   ttgtttgact atgctcaaga aaaaggtttt gccattccag ctatcaatgt cacttcatcc     120
   tcaactgttg ttgctgcttt agaagctgcc agagacaaca aggctccaat catcttgcaa     180
   acttctcaag gtggtgctgc ctactttgcc ggtaaaggtg tcgacaacaa agatcaagct     240
   gcttccattg ctggttcaat tgctgccgct cactacatta gagccattgc tccaacttat     300
   ggtatcccag ttgttttaca cactgatcac tgtgccaaaa aattattgcc atggtttgat     360
   ggtatgttga aagccgatga agaattcttt gctaagaccg gtactccatt gttctcatcc     420
   cacatgttgg atttatctga agaaaccgat gacgaaaaca ttgctacttg tgccaaatat     480
   ttcgaaagaa tggctaaaat gggtcaatgg ttagaaatgg aaattggtat cactggtggt     540
   gaagaagatg gtgtcaacaa cgaacacgtt gaaaaagatg ctttatacac ttctccagaa     600
   actgttttcg ctgtctacga atctttacac aagatttctc caaacttttc tattgctgct     660
   gcttttggta acgtccacgg tgtttacaaa ccaggtaatg tgcaattgag accagaaatc     720
   ttgggtgacc accaagttta cgctaagaaa caaattggta ctgatgctaa acacccatta     780
   tacttggttt tccacggtgg ttctggttct actcaagaag aattcaacac tgctatcaag     840
   aatggtgttg tcaaggtcaa cttggacact gattgtcaat atgcttactt gactggtatc     900
   agagattacg tcaccaacaa gattgaatac ttgaaagcac cagttggtaa cccagaaggt     960
   gctgacaaac caaacaagaa atactttgac ccaagagtct gggttagaga aggtgaaaag    1020
   accatgtcca agagaattgc tgaagctttg gatattttcc acaccaaagg acaattgtaa    1080
<210> 2
   <211> 359
   <212> PRT
   <213> Candida albicans
<220>
   <221> MISC_FEATURE
   <222> (1)..(359)
   <223> Fructose bisphosphate aldolase
<400> 2
   Met Ala Pro Pro Ala Val Leu Ser Lys Ser Gly Val Ile Tyr Gly Lys
   1               5                   10                  15
   Asp Val Lys Asp Leu Phe Asp Tyr Ala Gln Glu Lys Gly Phe Ala Ile
               20                  25                  30
   Pro Ala Ile Asn Val Thr Ser Ser Ser Thr Val Val Ala Ala Leu Glu
           35                  40                  45
   Ala Ala Arg Asp Asn Lys Ala Pro Ile Ile Leu Gln Thr Ser Gln Gly
       50                  55                  60
   Gly Ala Ala Tyr Phe Ala Gly Lys Gly Val Asp Asn Lys Asp Gln Ala
   65                  70                  75                  80
   Ala Ser Ile Ala Gly Ser Ile Ala Ala Ala His Tyr Ile Arg Ala Ile
                   85                  90                  95
   Ala Pro Thr Tyr Gly Ile Pro Val Val Leu His Thr Asp His Cys Ala
               100                 105                 110
   Lys Lys Leu Leu Pro Trp Phe Asp Gly Met Leu Lys Ala Asp Glu Glu
           115                 120                 125
   Phe Phe Ala Lys Thr Gly Thr Pro Leu Phe Ser Ser His Met Leu Asp
       130                 135                 140
   Leu Ser Glu Glu Thr Asp Asp Glu Asn Ile Ala Thr Cys Ala Lys Tyr
   145                 150                 155                 160
   Phe Glu Arg Met Ala Lys Met Gly Gln Trp Leu Glu Met Glu Ile Gly
                   165                 170                 175
   Ile Thr Gly Gly Glu Glu Asp Gly Val Asn Asn Glu His Val Glu Lys
               180                 185                 190
   Asp Ala Leu Tyr Thr Ser Pro Glu Thr Val Phe Ala Val Tyr Glu Ser
           195                 200                 205
   Leu His Lys Ile Ser Pro Asn Phe Ser Ile Ala Ala Ala Phe Gly Asn
   210 215 220
   Val His Gly Val Tyr Lys Pro Gly Asn Val Gln Leu Arg Pro Glu Ile
   225 230 235 240
   Leu Gly Asp His Gln Val Tyr Ala Lys Lys Gln Ile Gly Thr Asp Ala
   245 250 255
   Lys His Pro Leu Tyr Leu Val Phe His Gly Gly Ser Gly Ser Thr Gln
   260 265 270
   Glu Glu Phe Asn Thr Ala Ile Lys Asn Gly Val Val Lys Val Asn Leu
   275 280 285
   Asp Thr Asp Cys Gln Tyr Ala Tyr Leu Thr Gly Ile Arg Asp Tyr Val
   290 295 300
   Thr Asn Lys Ile Glu Tyr Leu Lys Ala Pro Val Gly Asn Pro Glu Gly
   305 310 315 320
   Ala Asp Lys Pro Asn Lys Lys Tyr Phe Asp Pro Arg Val Trp Val Arg
   325 330 335
   Glu Gly Glu Lys Thr Met Ser Lys Arg Ile Ala Glu Ala Leu Asp Ile
   340 345 350
   Phe His Thr Lys Gly Gln Leu
   355
<210> 3
   <211> 1905
   <212> DNA
   <213> Candida albicans
<220>
   <221> misc_feature
   <222> (1)..(1905)
   <223> Hyphal wall protein
<400> 3
   atgagattat caactgctca acttattgct atcgcttatt acatgttatc aattggggcc 60
   actgtcccac aggtagacgg tcaaggtgaa acagaggaag ctcttattca aaagagatct 120
   tatgattact atcaagaacc atgtgatgat tacccacaac aacaacaaca acaagagcct 180
   tgtgattacc cacaacaaca acagcaggaa gaaccttgtg attacccaca acaacaacca 240
   caagagccat gtgactatcc acaacagcca caagaacctt gtgactaccc acaacaacca 300
   caagaacctt gtgactaccc acaacaacca caagaacctt gcgacaatcc acctcaacct 360
   gatgttcctt gtgacaatcc tcctcaacct gatgttcctt gtgacaatcc tcctcaacct 420
   gatattcctt gtgacaatcc tcctcaacct gatattcctt gtgacaatcc tcctcaacct 480
   gatcagcctg atgacaatcc tcctattcca aacattccaa ccgattggat tccaaatatt 540
   ccaactgatt ggatcccaga tattccagaa aagccaacaa ctccagctac tactccaaac 600
   attcctgcta caactactac ttctgaatca tcatcttctt cttcttcttc atcatcatct 660
   actactccaa aaacttctgc ttcaactaca cctgaatctt ctgttccagc taccactcca 720
   aacacttctg ttccaacaac ttcttcagaa tcaactactc cagctactag cccagaaagt 780
   tctgttccag ttacttctgg atcatctatt ttagctacca cttcagaatc atcatctgct 840
   ccagctacta ctccaaatac atctgttcca accactacta ctgaagccaa atcatcaagt 900
   actccattaa ctactactac tgaacatgat acaactgttg tcactgttac ttcatgttct 960
   aacagtgttt gtaccgaaag tgaagttact actggtgtta ttgtcatcac atctaaagat 1020
   actatttaca ccacttactg tccattgact gaaactactc cagtttctac tgctccagcc 1080
   actgaaacac caactggtac agtatccact tctactgaac aatcaactac tgttattact 1140
   gttacttcat gttctgaaag ctcttgtacc gaatctgaag ttactactgg tgttgttgtt 1200
   gttacttctg aggaaactgt ctacactaca ttctgtccat tgactgaaaa cactccaggt 1260
   actgattcaa ctccagaagc ttccattcca cctatggaaa caattcctgc tggttcagaa 1320
   tcatccatgc ctgccggtga aacctctcca gctgttccaa aatcagatgt tccagctact 1380
   gaatcagctc cagttcctga aatgactcca gctggttcac aaccatctat tcctgccggt 1440
   gaaacctctc cagctgttcc aaaatcagat gttccagcta ctgaatctgc tcctgctcct 1500
   gaaatgactc cagctggtac tgaaactaaa ccagctgctc caaaatcatc agctcctgcc 1560
   actgaacctt ccccagttgc tccaggtact gaatccgcac cagctggtcc aggtgcttct 1620
   tcttctccaa aatcttctgt tttggctagt gaaacctcac caattgctcc aggtgctgaa 1680
   accgctccag ctggctcaag tggtgctatt actattccgg aatctagtgc tgtcgtctct 1740
   acgactgaag gtgctattcc aactacatta gaatcagttc cactcatgca accatctgcc 1800
   aattactcaa gtgtcgctcc tatttctaca tttgaaggtg ctggtaacaa catgagattg 1860
   actttcggtg ctgctattat tggtattgct gcattcttga tctaa 1905
<210> 4
   <211> 634
   <212> PRT
   <213> Candida albicans
<220>
   <221> MISC_FEATURE
   <222> (1)..(634)
   <223> Hyphal wall protein
<400> 4
   Met Arg Leu Ser Thr Ala Gln Leu Ile Ala Ile Ala Tyr Tyr Met Leu
   1               5                   10                  15
   Ser Ile Gly Ala Thr Val Pro Gln Val Asp Gly Gln Gly Glu Thr Glu
               20                  25                  30
   Glu Ala Leu Ile Gln Lys Arg Ser Tyr Asp Tyr Tyr Gln Glu Pro Cys
           35                  40                  45
   Asp Asp Tyr Pro Gln Gln Gln Gln Gln Gln Glu Pro Cys Asp Tyr Pro
       50                  55                  60
   Gln Gln Gln Gln Gln Glu Glu Pro Cys Asp Tyr Pro Gln Gln Gln Pro
   65                  70                  75                  80
   Gln Glu Pro Cys Asp Tyr Pro Gln Gln Pro Gln Glu Pro Cys Asp Tyr
                   85                  90                  95
   Pro Gln Gln Pro Gln Glu Pro Cys Asp Tyr Pro Gln Gln Pro Gln Glu
               100                 105                 110
   Pro Cys Asp Asn Pro Pro Gln Pro Asp Val Pro Cys Asp Asn Pro Pro
           115                 120                 125
   Gln Pro Asp Val Pro Cys Asp Asn Pro Pro Gln Pro Asp Ile Pro Cys
       130                 135                 140
   Asp Asn Pro Pro Gln Pro Asp Ile Pro Cys Asp Asn Pro Pro Gln Pro
   145                 150                 155                 160
   Asp Gln Pro Asp Asp Asn Pro Pro Ile Pro Asn Ile Pro Thr Asp Trp
                   165                 170                 175
   Ile Pro Asn Ile Pro Thr Asp Trp Ile Pro Asp Ile Pro Glu Lys Pro
               180                 185                 190
   Thr Thr Pro Ala Thr Thr Pro Asn Ile Pro Ala Thr Thr Thr Thr Ser
           195                 200                 205
   Glu Ser Ser Ser Ser Ser Ser Ser Ser Ser Ser Ser Thr Thr Pro Lys
       210                 215                 220
   Thr Ser Ala Ser Thr Thr Pro Glu Ser Ser Val Pro Ala Thr Thr Pro
   225                 230                 235                 240
   Asn Thr Ser Val Pro Thr Thr Ser Ser Glu Ser Thr Thr Pro Ala Thr
   245 250 255
   Ser Pro Glu Ser Ser Val Pro Val Thr Ser Gly Ser Ser Ile Leu Ala
   260 265 270
   Thr Thr Ser Glu Ser Ser Ser Ala Pro Ala Thr Thr Pro Asn Thr Ser
   275 280 285
   Val Pro Thr Thr Thr Thr Glu Ala Lys Ser Ser Ser Thr Pro Leu Thr
   290 295 300
   Thr Thr Thr Glu His Asp Thr Thr Val Val Thr Val Thr Ser Cys Ser
   305 310 315 320
   Asn Ser Val Cys Thr Glu Ser Glu Val Thr Thr Gly Val Ile Val Ile
   325 330 335
   Thr Ser Lys Asp Thr Ile Tyr Thr Thr Tyr Cys Pro Leu Thr Glu Thr
   340 345 350
   Thr Pro Val Ser Thr Ala Pro Ala Thr Glu Thr Pro Thr Gly Thr Val
   355 360 365
   Ser Thr Ser Thr Glu Gln Ser Thr Thr Val Ile Thr Val Thr Ser Cys
   370 375 380
   Ser Glu Ser Ser Cys Thr Glu Ser Glu Val Thr Thr Gly Val Val Val
   385 390 395 400
   Val Thr Ser Glu Glu Thr Val Tyr Thr Thr Phe Cys Pro Leu Thr Glu
   405 410 415
   Asn Thr Pro Gly Thr Asp Ser Thr Pro Glu Ala Ser Ile Pro Pro Met
   420 425 430
   Glu Thr Ile Pro Ala Gly Ser Glu Ser Ser Met Pro Ala Gly Glu Thr
   435 440 445
   Ser Pro Ala Val Pro Lys Ser Asp Val Pro Ala Thr Glu Ser Ala Pro
   450 455 460
   Val Pro Glu Met Thr Pro Ala Gly Ser Gln Pro Ser Ile Pro Ala Gly
   465 470 475 480
   Glu Thr Ser Pro Ala Val Pro Lys Ser Asp Val Pro Ala Thr Glu Ser
                   485                 490                 495
   Ala Pro Ala Pro Glu Met Thr Pro Ala Gly Thr Glu Thr Lys Pro Ala
               500                 505                 510
   Ala Pro Lys Ser Ser Ala Pro Ala Thr Glu Pro Ser Pro Val Ala Pro
           515                 520                 525
   Gly Thr Glu Ser Ala Pro Ala Gly Pro Gly Ala Ser Ser Ser Pro Lys
       530                 535                 540
   Ser Ser Val Leu Ala Ser Glu Thr Ser Pro Ile Ala Pro Gly Ala Glu
   545                 550                 555                 560
   Thr Ala Pro Ala Gly Ser Ser Gly Ala Ile Thr Ile Pro Glu Ser Ser
                   565                 570                 575
   Ala Val Val Ser Thr Thr Glu Gly Ala Ile Pro Thr Thr Leu Glu Ser
               580                 585                 590
   Val Pro Leu Met Gln Pro Ser Ala Asn Tyr Ser Ser Val Ala Pro Ile
           595                 600                 605
   Ser Thr Phe Glu Gly Ala Gly Asn Asn Met Arg Leu Thr Phe Gly Ala
       610                 615                 620
   Ala Ile Ile Gly Ile Ala Ala Phe Leu Ile
   625                 630

## Description

The present invention concerns an *in vitro* method for predicting or diagnosing invasive candidiasis (IC) in a subject by the determination of the presence of a fructose biphosphate aldolase 1 (Fba1) protein. The invention also relates to a method of determining a suitable treatment regimen for a patient and to a kit for carrying out the methods described herein.

Candidiasis is a fungal infection with yeast(s) of any Candida species, of which *Candida albicans* is the most common. Candidiasis encompasses infections that range from superficial infections of skin and mucosal membranes, which cause local inflammation and discomfort, to systemic or invasive candidiasis (IC). Candida infections of the latter category are also referred to as deep-seated tissue infection affecting one or multiple organ(s) revealed or not by blood culture (candidemia). This severe forms of the disease are usually confined to severely immunocompromised persons, such as cancer, transplant, and AIDS patients, but may occur also in medical and surgical intensive care units and intravenous catheter bearing patients.

Invasive candidiasis remains a problem of public health persisting in medical and surgical intensive care units, onco-haematology, bone marrow and hematopoietic stem cell transplantation and premature newborns wards. According to recent reports *Candida spp.* rank as the fourth most common cause of nosocomial bloodstream infections and are characterized by an important medical and economic impact linked to difficulties of the clinical and biological diagnosis. Systemic candidiasis is associated with long hospital stays and mortality rates of 18 to 70% and a shift in the spectrum of infecting species has also occurred. Non*-Candida albicans* species, although they may have a lower intrinsic pathogenic potential than *C. albicans,* are now identified in more than 45-60% of episodes of hematogenously disseminated candidiasis. Moreover, the increased morbidity as a consequence of candidemia generates important extra costs related to longer hospital stays and prophylactic, empirical, or curative antifungal treatment. While the availability of new antifungal drugs such as echinocandins and new azoles is a promising step toward improving outcomes for patients with invasive candidiasis, a definitive and early diagnostic approach is imperative to avoid delay in the initiation of treatment for infected patients and to prevent unnecessary therapy in high-risk individuals who are only colonized. This delay in the initiation of appropriate antifungal treatment is a cause for the high morbi-mortality rates of IC and is related to a lack of rapid and reliable tests discriminating patients with IC from patients with superficial colonization.

Histological examination of biopsies and blood culture are still considered as the "gold standard" for the diagnosis of IC; however these methods are rarely performed or poorly sensitive, respectively.

Several rapid, non-culture based methods have been developed including the detection of anti*-Candida* antibody against immunogenic targets and the detection of circulating Candida antigen (Yeo and Wong. Clin Microbiol Rev 2002;15:465-84). However, only a few assays have been standardized and are commercially available. The antigen assays target the detection of mannan (Weiner and Yount. J Clin Invest 1976;58:1045-53; Herent et al. J Clin Microbiol 1992;30:2158-64), D/L arabinitol (Switchenko et al. J Clin Microbiol 1994;32:92-7), heat labile antigen (Fung et al. J Clin Microbiol 1986;24:542-7) and (1→3)-β-D-glucans (Ostrosky-Zeichner et al. Clin Infect Dis 2005;41:654-9; Senn et al. Clin Infect Dis 2008;46:878-85). Another assay targeting Enolase has been abandoned (Walsh TJ et al. N Engl J Med. 1991 Apr 11;324(15):1026-31). Antibody assays target the mannan, the major antigenic component of the yeast cell wall. However, these non-culture methods, even helpful, exhibit numerous drawbacks in their sensitivity and specificity, and sometimes require the combination of two or more assay results for accurate diagnosis of IC.

Improvement of specificity has been obtained by combining the detection of serum mannan and anti-mannan antibodies (Sendid et al. J Clin Microbiol 1999;37:1510-7). Such an improvement can be explained by the fact that when a specific antigen and antibodies to this antigen are detected in a same patient, the antibodies may facilitate the clearance of the antigen. A meta-analysis study of diagnostic values of these two assays has been published (Mikulska et al. Crit Care. 2010;14(6):R222). The use of mannan antigen and anti-mannan antibodies in the diagnosis of invasive candidiasis: recommendations from the Third European Conference on Infections in Leukemia. Detection of mannan appears to be very specific: 93% on 767 patients (11 studies) with a thin 95% IC (91%-94%). However the sensitivity is weak: 58% on 453 patients from 14 studies (95% IC: 53%-62%). Addition of the detection of anti-mannan antibodies allowed to increase the sensitivity up to 83% (95% IC: 79%-87%) but lowered the specificity to 72% due to the lower specificity of anti mannan antibodies detection especially in heavily colonized patients (Ellis et al. J Med Microbiol 2009;58:606-15).

Many additional proteins that could help in the diagnosis of IC have been pointed out during the past 10 years especially with the development of proteomics analysis. Some of them seem to be specific to pathogenic process. Indeed, more than twenty Candida proteins have been reported to be over expressed during the switch saprophytic/pathogenic phases leading to investigate their values for the diagnosis of IC (Clancy et al. J Clin Microbiol 2008; 46:1647-54). The diagnostic value has not been confirmed for the majority of these identified proteins and only primary results on non-standardized or sophisticated assay have been obtained for the others. Hyphal Wall Protein (Hwp1) [Staab et al. J Biol Chem 1996;271:6298-305; Lain et al. BMC Microbiol 2007;7:35 ; Martin et al. Int J Med Microbiol;301:417-22], Agglutinin like sequence family (Als3) [Martin et al. Int J Med Microbiol;301:417-22 ; Hoyer et al. Curr Genet 1998;33:451-9 ; Green et al. Infect Immun 2005;73:1852-5], Superoxide dismutase (Sod) [Martin et al. Int J Med Microbiol;301:417-22], Methionine synthetase -Met6 [Pitarch et al. Proteomics 2001;1:550-9 ; Pitarch et al. Proteomics 2004;4:3084-106 ; Pitarch et al. Proteomics Clin Appl 2007;1:1221-42], Malate deshydrogenase [Hernando et al. Int Microbiol 2007;10:103-8], Fructose biphosphate aldolase [Pitarch et al. Proteomics 2001;1:550-9 ; Hernando et al. Int Microbiol 2007;10:103-8; Li et al. BMC Infectious Diseases 2013;13:253], Hsp70 family [La Valle et al. Infect Immun 2000;68:6777-84; Pitarch et al. Proteomics 2001;1:550-9], Hsp90 [Matthews RC. J Med Microbiol 1992;36:367-70; Pitarch et al. Proteomics 2001;1:550-9], Phosphoglycerate kinase (PGK) [Pitarch et al. Proteomics 2001;1:550-9 ; Pitarch et al. Proteomics 2004;4:3084-106 ; Hernando et al. Int Microbiol 2007;10:103-8 ; Clancy et al. J Clin Microbiol 2008;46:1647-54], Diacylglycerol kinase catalytic domain [Hernando et al. Int Microbiol 2007;10:103-8], Glyceraldehyde 3-phosphate dehydrogenase (G3P) [Pitarch et al. Proteomics 2001;1:550-9], Alcohol Dehydrogenase (ADH1) [Pitarch et al. Proteomics 2001;1:550-9], Diacylglycerol kinase [Hernando et al. Int Microbiol 2007;10:103-8], 65 kDa mannoprotein (CamP65) [Berzaghi et al. Clin Vaccine Immunol 2009;16:1538-45], Enolase 1 (Eno1) [Li et al. BMC Infectious Diseases 2013;13:253] make part of all these proteins identified by proteomic approaches.

Limited results are available to conclude on the possibility to use these proteins for improving the diagnosis of IC and further prospective studies are required to confirm the usefulness in clinical practice.

In particular, the Fba1 protein has been identified as a protein which expression is induced in hyphae forming *Candida albicans* strain Sc5314 when cultured *in vitro* (patent application US 2004/0014061). The ability to form hyphae is regarded as a virulence factor because these structures are often observed invading tissue, and strains that are unable to form hyphae are defective in causing infection. The presence of anti-Fba1 antibodies in the serum of patients with invasive candidemia was described previously leading to the suggestion that IgG against Fba1 would have diagnostic value for candidemia [Hernando et al. Int Microbiol 2007;10:103-8; Li et al. BMC Infectious Diseases 2013;13:253].

Yet, that antibodies against Candida Fba1 may have diagnostic value is not indicative or predictive that Candida Fba1 protein per se would also have diagnostic value. In particular, the presence of circulating Fba1 protein has never been reported in the serum of patients with invasive candidemia. Furthermore, for a pathogen antigen to be relevant for clinical diagnostic, the minimum requirements are that the antigen be released in the circulation and that it be stable therein in order to remain detectable. In this respect, it is observed that in patent application US 2004/0014061, Fba1 protein expression was demonstrated by Nothern blot analysis and 2D gel electrophoresis of protein extracts from *in vitro* cultured *Candida albicans* strains, i.e. in conditions that cannot not be predictive of a concrete diagnostic value of *Candida* Fba1 protein.

The inventors evaluated the diagnostic performances of one ELISA tests detecting circulating *Candida spp* Fba1 protein by capture with a specific monoclonal antibody directly obtained by screening on Fba1 recombinant protein generated by culture of *E*. *coli* transfected by a specific plasmid containing fructose biphosphate aldolase 1 (Fba1) *Candida albicans* gene. This evaluation of diagnostic potential was performed in comparison with Platelia™ *Candida* Ag plus (current commercialized antigen diagnosis test of IC) on a cohort consisted of patients with proved IC determined by positive blood culture by *Candida albicans* (29 patients and 29 sera) or Candida non albicans species (21 patients and 21 sera). Control group consisted of 55 blood donors (55 sera).

These investigations have shown usefulness of Fba1 antigen for the diagnostic of IC.

With a specificity fixed arbitrarily at 100%, Fba1 antigen has shown a specific sensibility, positive predictive value (PPV), negative predictive value (NPV) and accuracy of 42%, 100%, 72.4% and 79.7%, respectively while mannanemia lead to a sensitivity, PPV, NPV and accuracy of 38%, 100%, 70.5% and 77.5%, respectively. Performances of these 2 biomarkers are also similar in term of statistical parameters.

However, to implement mannanemia determination, serum samples require to be denatured, for instance with an EDTA treatment solution and by heating at 100°C, followed by centrifugation. This pretreatment of the samples renders automation of the assay impossible. Contrary to mannanemia determination, Fba1 antigen detection does not require sample treatment and can be performed on a low volume of biological sample.

Furthermore, the precocity of diagnosis and the volume of biological sample being a key element of IC clinical management and coverage of patients, the gain of time and the decrease of sample volume needed bring an important practicability of this new assay in comparison to current diagnosis test.

### Method for improving the diagnosis of invasive candida infections by Fba1 protein detection

The invention relates to an *in vitro* method for diagnosing invasive candidiasis (IC) in a subject, said method comprising the step of:
a) detecting the presence of a Candida Fba1 protein in a (first) blood, plasma or serum sample of the subject, and
b) determining that the subject has IC based upon the presence of said Candida Fba1 protein in said blood, plasma or serum sample of the subject.

"Invasive candidiasis" or "IC" is also called candidemia and denotes systemic infection with yeast(s) of any Candida species, e.g. *Candida albicans, Candida parapsilosis, Candida kruseï, Candida tropicalis, Candida glabrata*, *Candida lusitaniae, Geotrichum capitatum, Candida norvegiensis, and Candida guillermondii. Candida albicans* is the most common Candida species. IC may be ultimately diagnosed by histological examination of biopsies and detection of Candida species in blood cultures.

Preferably, invasive candidiasis is due to infection with a Candida species selected from the group consisting of *Candida albicans, Candida parapsilosis, Candida kruseï, Candida tropicalis, Candida glabrata, Candida lusitaniae, Geotrichum capitatum, and Candida norvegiensis.*

As used herein, "Candida Fba1", "Candida Fba1 protein", or "Fba1" denotes the fructose-1,6-bisphosphate aldolase (Fba1) which catalyzes the reversible cleavage of fructose-1,6-bisphosphate to dihydroxyacetone phosphate and glyceraldehyde 3-phosphate. Candida Fba1 is a cytoplasmic enzyme involved in glycolysis and gluconeogenesis and which is regulated by yeast-hyphal switch. Fba1 is a unique gene in *C. albicans* (http://genolist.pasteur.fr/CandidaDB). Fba1 protein from *Candida albicans* is encoded by a polynucleotide comprising sequence SEQ ID NO:1, and consists of a 360 amino acid long sequence as shown in SEQ ID NO:2. These sequences were published in Jones et al. Proc. Natl. Acad. Sci. U.S.A. 101 (19), 7329-7334 (2004).

A "subject" or "patient" may be a human or a non-human mammal, such as monkeys, dogs, cats, guinea pigs, hamsters, rabbits, cows, horses, goats and sheep. Preferably, the subject is a human, in particular a man, a woman or a child (0-18 year old).

By "detecting the presence" of an antigen (protein) or antibody, it is meant that in the largest extent it is determined if the antigen or antibody is present or absent in the sample to be analyzed. According to an embodiment, the level of said Candida antigen and/or antibody directed against said Candida protein is detected.

The presence or level of a Candida Fba1 protein in a blood, plasma or serum sample of the subject is representative of the presence or level of the Candida Fba1 protein in circulating blood of the subject. The presence or level of Candida Fba1 protein may denote in particular the presence or level of soluble Candida Fba1 protein.

Accordingly, the method may comprise the step consisting of detecting the level of Candida Fba1 protein in a blood, plasma or serum sample of the subject, wherein an elevated level of said Candida Fba1 protein, relative to a reference level is indicated of invasive candidiasis.

In an advantageous embodiment of the method according to the invention, the method further comprises the steps of detecting the presence of antibodies directed against Candida hyphal wall protein (Hwp1) in a second blood or plasma sample of said subject, and determining that the subject has IC based upon the presence of antibodies directed against Candida Hwp1 protein . Thus, according to said method, IC is diagnosed when either one of Fba1 protein or anti-Hwp1 antibody is detected in the analysed first and second samples.

In a particular aspect of this embodiment, the method of the invention comprises the step of detecting the level of antibodies directed against Candida Hwp1 protein in the blood, plasma or serum sample, wherein an elevated level of said antibodies directed against Candida Hwp1 protein relative to a reference level is indicated of invasive candidiasis.

"Hyphal wall protein" or "Hwp1" denotes a mannoprotein specifically expressed in the cell wall surface of the hyphae of *C. albicans.* A representative sequence for Hwp1 is Hwp1 from *Candida SC5314* which is encoded for instance by a polynucleotide comprising sequence SEQ ID NO:3, and which consist of the amino acid sequence SEQ ID NO:4. These sequences were published in Jones et al. Proc. Natl. Acad. Sci. U.S.A. 101 (19), 7329-7334 (2004). The Candida Hwp1 proteins can elicit antibodies in subjects infected with Candida species and developing IC.

It is to be understood that Candida Fba1 and Hwp1 proteins are intended to encompass any naturally existing variant or isoform protein differing from the respective representative amino acid sequences identified above by modification of the amino acid sequence and/or of the glycosylation pattern of the protein. By modification of the amino acid sequence it is meant deletion, addition or substitution, of at least one amino acid, for instance by 1 to 15 amino acids, such as by 1, 2, 3, 4, 5, 6, 7, 8, 9 or 10 amino acids. Preferably the variant or isoform protein has at least 80, 85, 87, 90, 91, 92, 93, 94, 95, 96, 97, 98, or 99% sequence identity with the representative amino acid sequence, as can be determined by global pairwise alignment using the Needleman-Wunsch algorithm. The percentage of sequence identity can be readily determined for instance using the program Needle, with the BLOSUM62 matrix, and the following parameters gap-open=10, gap-extend=0.5.

The presence or level of Candida Fba1 protein and of antibodies directed against Candida Hwp1 protein may be detected (i) in one and the same blood, plasma or serum sample of the patient (said first and second samples are thus a same or aliquots from a same sample) or (ii) in several blood, plasma or serum samples sequentially obtained from the same patient, (iia) either at the same time (i.e. essentially simultaneously or no more than 3 minutes apart) or at least no more than 72 hours, no more than 48 hours, no more than 24 hours, preferably no more than 12 hours, preferably no more than 6 hours, still preferably no more than 3 hours apart, or (iib) in the course of patient monitoring, e.g. 1, 2, 3, 4, 5, 6, 7, 8, 9, 10, 11, 12, 13 or 14 days apart.

In particular, detection of Candida Fba1 protein and antibodies directed against Candida Hwp1 protein can be conveniently performed simultaneously in a same sample as Fba1 detection does not required pre-treatment as otherwise required with mannanemia.

The method of the invention is preferably carried out at least twice a week. The method of the invention is also preferably carried out as long as the subject is at risk of developing invasive candidiasis, in particular as long as the patient is hospitalised.

### Immunoassay methods

Immunoassays are antibody-based methods which enable for measuring antigens or antibodies. They typically include indirect, sandwich, or competition immunoassays, for instance in a radioimmunoassay (RIA) or EIA (Enzyme ImmunoAssay) format.

A sandwich immunoassay is a method using two antibodies specific for the antigen to be detected, which bind to different sites on the antigen or ligand. The primary antibody (or capture antibody), which recognizes the antigen, is attached to a solid surface. The antigen is then added followed by addition of a second antibody referred to as the detection antibody. The detection antibody binds the antigen to a different or a repeated epitope(s). As the antigen concentration increases the amount of detection antibody increases leading to a higher measured response.

To quantify the extent of binding different reporters can be used: a radioactive tracer in RIA, or a fluorophore or enzyme in EIA (in particular in ELISA). In ELISA, an enzyme is typically attached to the detection antibody or to a third antibody which binds the detection antibody and which must be generated in a different species than detection antibodies (i.e. if the detection antibody is a rabbit antibody than the third antibody would be an anti-rabbit from goat, chicken, etc., but not rabbit). The substrate for the enzyme is added to the reaction that forms a colorimetric readout as the detection signal. The enzyme step of the assay can be replaced with a fluorophor-tagged detection and the fluorescent signal is measured in a fluorescent plate reader. The signal generated is proportional to the amount of target antigen present in the sample. Typically, a detection antibody conjugated to peroxidase and tetramethyl benzidine (TMB) as a chromogenic substrate may be used for detection in a sandwich immunoassay.

A competitive binding assay is based upon the competition of labelled and unlabelled antigen for a limited number of antibody binding sites. A fixed amount of labelled antigen and a variable amount of unlabelled antigen are incubated with an antibody bound to a solid phase. The amount of labelled antigen is a function of the total concentration of labelled and unlabelled antigen. As the concentration of unlabelled antigen is increased, less labelled antigen can bind to the antibody and the measured response decreases. Thus the lower the signal, the more unlabelled antigen there is in the sample.

In indirect immunoassay, an antigen is coated onto a solid surface. The coated solid surface is then incubated with a sample to analyse if it contains antibodies specific for the antigen. Detection of antigen-bound antibodies is then performed using a detectably labelled secondary antibody which binds to the antibody specific for the antigen. The secondary antibody may be labelled with a fluorophore which generates a fluorescent signal measurable in a fluorescent plate reader, or with an enzyme which can produce a colorimetric signal upon addition of the enzyme substrate.

The immunoassay may be performed in a lateral flow immunochromatographic assay format, high throughout and/or multiplex immunoassay format such as Luminex®, platform or any microfluidic derived format such as microchip immunoassays, etc...

As used herein, "antibody" or "immunoglobulin" have the same meaning, and are used equally. In natural conventional antibodies, two heavy chains are linked to each other by disulfide bonds and each heavy chain is linked to a light chain by a disulfide bond. There are two types of light chain, lambda (λ) and kappa (κ). There are five main heavy chain classes (or isotypes) which determine the functional activity of an antibody molecule: IgM, IgD, IgG (such as IgG₁, IgG₂, IgG₃ or IgG₄), IgA and IgE. The light chain includes two domains, a variable domain (VL) and a constant domain (CL). The heavy chain includes four domains, a variable domain (VH) and three constant domains (CHI, CH2 and CH3, collectively referred to as CH). The variable regions of both light (VL) and heavy (VH) chains determine binding recognition and specificity to the antigen. The specificity of the antibody resides in the structural complementarity between the antibody combining site and the antigenic determinant (epitope). Antibody combining sites are made up of residues that are primarily from the hypervariable or complementarity determining regions (CDRs). Occasionally, residues from non hypervariable or framework regions (FR) influence the overall domain structure and hence the combining site. The CDR refers to amino acid sequences which together define the binding affinity and specificity of the natural Fv region of a native immunoglobulin binding site. The light and heavy chains of an immunoglobulin each have three CDRs, designated L-CDR1, L-CDR2, L-CDR3 and H-CDR1, H-CDR2, H-CDR3, respectively. An antigen-binding site ("paratope"), therefore, includes six CDRs, comprising the CDR set from each of a heavy and a light chain V region. FR refers to amino acid sequences interposed between CDRs. Antibodies may be monoclonal antibodies (mAb), polyclonal antibodies, or fragment thereof, but also bispecific or multispecific antibodies. Monoclonal antibodies are monospecific antibodies which bind to the same epitope on an antigen. Monoclonal antibodies may be produced by clones of a hybridoma cell line or by recombinant technologies. Polyclonal antibodies are a combination of immunoglobulins directed against a specific antigen, each immunoglobulin possibly binding to a different epitope on the antigen. Polyclonal antibodies are generally produced by immunisation of a suitable mammal, such as a mouse, rabbit or goat. Antibody fragments include for instance Fab, Fab', F(ab')₂, Fv, scFv, and nanobodies (single chain antibodies). Bispecific antibodies are bivalent recombinant antibodies composed of fragments of two different monoclonal antibodies and consequently can bind to two different types of antigen, or to two different molecules or epitopes of a same antigen. Multispecific antibodies are multivalent recombinant antibodies composed of fragments of more than two different monoclonal antibodies.

In the method of the invention, Candida Fba1 protein is preferably detected by immunoassay, preferably a sandwich immunoassay.

For the detection of a Candida Fba1 protein, an antibody binding preferentially or specifically to said Candida Fba1 protein may be used. The antibodies (capture and/or detection antibodies) which may be used for the detection of Candida Fba1 protein are preferably (monovalent) monoclonal antibodies, or fragment thereof, in particular Fab antibodies, directed against Candida Fba1 protein.

Indeed, in initial attempts to detect circulating Candida Fba1 protein in the serum of patients with IC or of control subjects, the inventors used bispecific antibodies against Fba1 in order to have increased avidity for the protein to be detected. However, while 18 out of 19 patients' sera showed significant level of circulating Fba1 protein, the use of bivalent antibodies caused significant background noise and led to an elevated percentage of false-positive in control samples (58.5%). Switching to monovalent antibodies made it possible to greatly reduce non-specific binding in control samples.

As used herein, an antibody binding "preferentially" to said Candida Fba1 protein, is an antibody showing less that 15% cross reactivity, preferably less than 10% or 5% cross reactivity, with an antigen different from said Candida glycan. An antibody binding "specifically" to said Candida Fba1 protein is an antibody showing no cross-reactivity with any other antigen.

According to the invention, antibodies directed against Candida Fba1 protein can be antibodies which have been raised and/or selected against a recombinant Fba1 protein expressed in *E. coli.*

Antibodies directed against Candida Fba1 protein are commercially available, for instance from Signalway Antibody LLC (catalog No M102), or may readily be obtained by the one skilled in the art by known methods of immunization/selection, or by selection of antibodies against Fba1 within antibody libraries such as Human Combinatorial Antibody Library (HuCAL technology developed by AbDSerotec).

In the method of the invention, antibodies directed against Candida Hwp1 protein are preferably detected by immunoassay.

For the detection of antibody(ies) directed against Candida Hwp1 protein, purified Hwp1 Candida protein, or epitopic fragments thereof, or a polypeptide comprising full length protein, epitopic fragments or variants thereof which have been recombinantly produced or chemical synthesized may be used. When the Hwp1 Candida protein is a glycoprotein, the purified Hwp1 Candida protein, or epitopic fragments thereof, may have been deglycosylated. Purified and recombinant full length proteins or epitopic fragments thereof may or may not include glycosylation(s). Preferably the polypeptide used for detection of an antibody directed against Hwp1 Candida protein is devoid of glycosylation.

While full length Hwp1 protein can be used for detecting anti-Hwp1 antibodies, it has been reported that a 161 amino acid long N-terminal fragment thereof (amino acids at positions 41 to 200) increased antibody detection by ELISA in sera of patients with invasive candidiasis (Lain et al., 2007, BMC Microbiology, 7:35). Furthermore, a fragment of Hwp1 consisting of amino acids at positions 27 to 103 of Hwp1 was shown to be an epitopic fragment of Hwp1 (Fradin et al., 2008 Infect. Immun. 76, 4509-4517).

Accordingly, a polypeptide which may be used for detecting anti-Hwp1 antibodies may be selected in particular from the group consisting of a polypeptide comprising, or consisting of :
a) SEQ ID NO:4 or a naturally existing variant or isoform thereof;
b) an epitopic fragment of a polypeptide defined in a), in particular a fragment consisting of amino acids at positions 41 to 200, or 27 to 203 of SEQ ID NO:4, or a variant thereof.

As used herein, the term "epitopic fragment" refers to a polypeptide fragment of a protein which consists of a stretch of contiguous amino acids of the protein and comprises one or more epitopes of said protein. An epitopic fragment retains the capacity to be bound by at least part, preferably all, of the antibodies that bind to the full length protein from which the fragment is derived. An epitope may be linear when made of contiguous amino acid residues, or conformational when an antibody binds to discontinuous amino acids that come together in three dimensional conformation. It is generally admitted that a linear epitope is constituted by at least 4 or 5 amino acid contiguous residues. Accordingly, an "epitopic fragment" according to the invention preferably comprises at least 4, 5, 6, 7, 8, 9, 10, 12, 15, 20, 25, 30, 40, 50 or 100 contiguous amino acids of the protein from which the epitopic fragment derives. An "epitopic fragment" may comprise less than 300, 250, 200, 150, 100, 50, 40, 30, 20 or 15 amino acids.

The fragments can be generated for instance by processing the full length protein, or a longer fragment of said protein, with a proteolytic enzyme (classically trypsin, chymotrypsin, or collagenase), or by chemical treatment with cyanogen bromide (CNBr), for instance.

Variant polypeptides of the full length Candida Hwp1 protein or of an epitopic fragment thereof may be obtained by modification of its amino acid sequence by deletion, addition or substitution, of at least one amino acid, for instance by 1 to 15 amino acids, such as by 1, 2, 3, 4, 5, 6, 7, 8, 9 or 10 amino acids. Preferably the variant polypeptide has at least 80, 85, 87, 90, 91, 92, 93, 94, 95, 96, 97, 98, or 99% sequence identity with the full length Candida protein or epitopic fragment thereof, as can be determined by global pairwise alignment using the Needleman-Wunsch algorithm, in particular using the program Needle, with the BLOSUM62 matrix, and the following parameters gap-open=10, gap-extend=0.5. A variant polypeptide retains the capacity to be bound by at least part, preferably all, of the antibodies that bind to the full length protein or the fragment thereof from which it derived. A variant polypeptide may typically comprise a heterologous polypeptide sequence, such as a tag (His-tag, GFP, etc...) to facilitate purification.

For epitopic fragments preferably containing no more than 20 amino acids, chemical synthesis such as solid phase synthesis may be employed for preparing the epitopic fragments.

In a particular embodiment of the method of the invention, Candida Fba1 protein and antibodies directed against Candida Hwp1 protein are detected in two separate immunoassays or in a single immunoassay.

### Diagnosis of invasive candidiasis

According to an embodiment, diagnosing invasive candidiasis may be performed by comparing the level of said Candida Fba1 protein with a reference level. If an elevated level of said Candida Fba1 protein, relative to their respective reference level is detected, then the subject is developing or has developed invasive candidiasis.

Diagnosing invasive candidiasis may also be performed by comparing the level of antibodies directed against Candida Hwp1 protein with a reference level. If an elevated level of said antibodies directed against Candida Hwp1 protein, relative to their respective reference level is detected, then the subject is developing or has developed invasive candidiasis.

The reference level(s) may be determined as a single value or a range of values which is determined based on the level of said Candida Fba1 protein or antibodies directed against Candida Hwp1 protein, as appropriate, measured in a population of healthy subjects, or in a population of subjects suffering from invasive candidiasis.

The reference level can also be determined by analysing a sample from the same subject for instance at an earlier time point prior to onset of invasive candidiasis or prior to suspicion of invasive candidiasis.

Comparison with a reference level may also be performed by comparing the level of said Candida Fba1 protein or of antibodies directed against Candida Hwp1 protein, as appropriate, measured in a standard sample constituted by a pool of sera obtained from patients having invasive candidiasis.

The reference level for a given marker may vary depending on the method used for measuring.

Based on the analysis of a reference set of blood, plasma or serum samples from subjects with invasive candidiasis and subjects without invasive candidiasis, a Relative Operating Characteristic (ROC) curve can be generated for each marker analysed. A ROC curve is a graphical representation of the sensitivity (or true positive rate) against the false positive rate (i.e. [1 - specificity], specificity being the true negative rate) of a marker-based test. A ROC space is defined by sensitivity and (1-specificity) as x and y axes respectively. The best possible prediction method would yield a point in the upper left corner or coordinate (0,1) of the ROC space, representing 100% sensitivity (no false negatives) and 100% specificity (no false positives). A completely random guess would give a point along a diagonal line (the so-called line of no-discrimination) from the left bottom to the top right corners. The diagonal divides the ROC space. Points above the diagonal represent good classification results (better than random), points below the line poor results (worse than random). The Area Under the Curve (AUC) of a ROC curve may be calculated. The higher the AUC, the higher the diagnostic accuracy of the diagnostic marker.

### Method for determining a suitable treatment regimen

The high rates of mortality associated with invasive candidiasis (IC) are mainly due to the delay in identifying that a subject is affected with IC and initiating the appropriate antifungal treatment.

The invention further relates to a method for determining a treatment regimen in a subject, said method comprising:
a) diagnosing if the subject has invasive candidiasis by carrying out the method as described above, and
b) if the subject is diagnosed as having invasive candidiasis, determining an antifungal treatment for said subject.

The invention also relates to a method for treating invasive candidiasis in a subject in need thereof, said method comprising:
a) diagnosing that the subject has invasive candidiasis by carrying out the method method as described above, and
b) administering an antifungal treatment to said subject.

The invention further relates to an antifungal treatment for use for treating invasive candidiasis in a subject, wherein said subject has been diagnosed with invasive candidiasis by carrying out the method as described above. In particular, the invention relates to an antifungal treatment for use for treating invasive candidiasis in a subject, comprising diagnosing invasive candidiasis in said subject by carrying out the method as described above.

The subject may be a subject suspected of having invasive candidiasis or at risk of invasive candidiasis. The subject may be in particular a subject having superficial infection of skin and/or mucosal membranes. The subject may also be for instance an immunocompromised subject bearing intravenous catheter.

An antifungal treatment may be for instance treatment with echinocandins which notably include Caspofungin (1-[(4R,5S)-5[(2-aminoethyl)amino]-N2-(10,12-dimethyl-1-oxotetradecyl)-4-hydroxy-L-ornithine]-5-[(3R)-3-hydroxy-L-ornithine]pneumocandin B₀), Micafungin and/or Anidulafungin (see for instance Patterson TF. Curr Infect Dis Rep 2006;8:442-8). Echinocandins are synthetically modified lipopeptides which inhibit the synthesis of (1,3)-β-D-glucan. Another antifungal treatment may be for instance treatment with Enfumafungin and Enfumafungin derivatives such as described in the patent application published as US 20110224228. Amphotericin B and its lipidic formulations, or antifungal azole derivatives such as fluconazole, itraconazole, and Voriconazole (see for instance Pfaller et al. J Clin Microbiol 2006;44:819-26 ; Mean et al. Crit Care 2008;12:204) may also be used.

Accordingly, in some embodiments, the antifungal treatment is selected from the group consisting of echinocandins, enfumafungin, enfumafungin derivatives, amphotericin B, lipidic formulations of amphotericin B, and antifungal azole derivatives. In an embodiment, the echinocandins is selected from the group consisting of caspofungin micafungin and anidulafungin; In an embodiment, an antifungal azole derivative is selected from the group consisting of fluconazole, itraconazole, and voriconazole.

Combined administration of these compounds, or combination with other active principles is possible.

The treatment is preferably administered as long as circulating mannan can be detected in the serum of the subject.

### Kit for diagnosing invasive candidiasis

The invention further relates to a kit for diagnosing invasive candidiasis comprising, or consisting of means for determining the presence or level of a Candida Fba1 protein, and means for determining the presence or level of antibodies directed against Candida Hwp1 protein.

Means for determining the presence or level of a Candida Fba1 may be in particular antibodies such as those described above. Such means may also comprise a chromogenic substrate and enzyme(s).

Means for determining the presence or level of antibody directed against a Candida Hwp1 protein may comprise purified Candida protein, or epitopic fragments thereof, or a polypeptide comprising full length protein, epitopic fragments or variants thereof which have been recombinantly produced or chemical synthesized, as disclosed above.

In an embodiment, the kit further comprises a solid support for the immobilization of proteins. Appropriate materials for immobilization of proteins include glass, silicon dioxide, nylon, acryl amide derivatives, silica materials, polymeric materials, such as fluoropolymers, metal oxides, etc. These substrate materials may have been pretreated before immobilization of proteins, for example, with silane coupling agents.

The kit can further comprises a positive sample, i.e. at least one blood, plasma or serum sample of a subject with invasive candidiasis, and/or a control sample, i.e. at least one blood, plasma or serum sample of a healthy subject.

Although having distinct significances, the terms "comprising", "containing", and "consisting of" are used in an interchangeable way in the description of the invention, and can be replaced one by the other.

The invention will be further illustrated in view of the following figures and examples.

### FIGURES

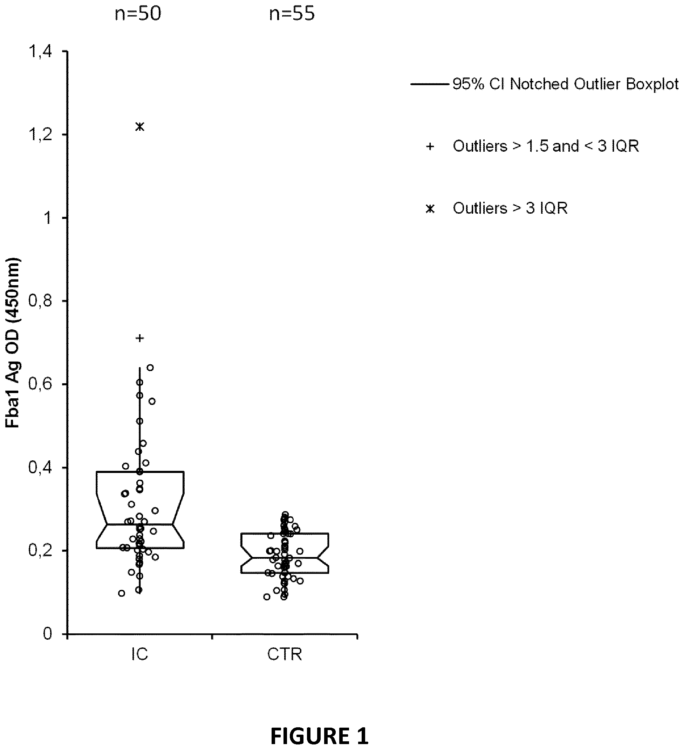

Figure 1. Boxplot representation of diagnosis potential of Candida Fba1 protein. Boxplot representation is a convenient way of graphically depicting groups of numerical data through their five-number summaries (the smallest observation, lower quartile (Q1), median (Q2), upper quartile (Q3), and largest observation). Boxplots can be useful to display differences between populations without making any assumptions of the underlying statistical distribution. CTRL represents controls and IC represents invasive candidiasis patients.

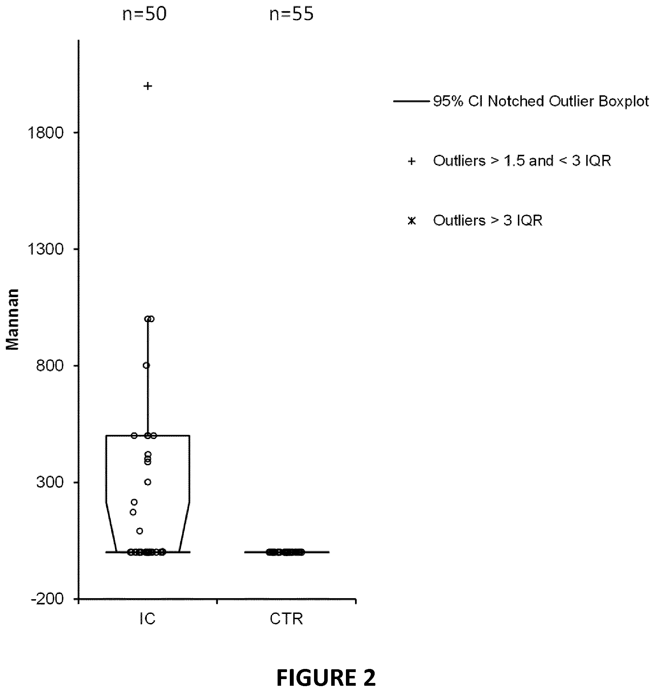

Figure 2. Boxplot representation of diagnosis potential of Candida Mannan antigen.

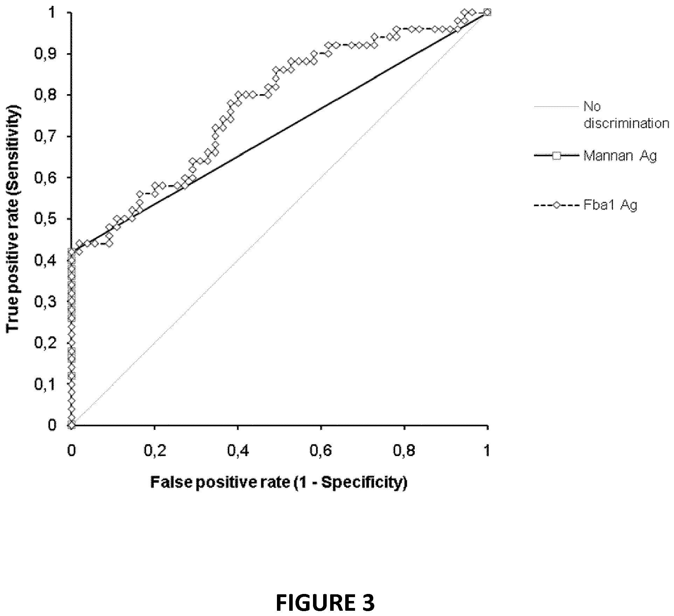

Figure 3. ROC curves showing the diagnostic performances of Candida Fba1 protein (diamonds) And of the commercially available mannan (antigen) detection test (squares).The diagonal doted-line corresponds to the random-guess or no-discrimination line.

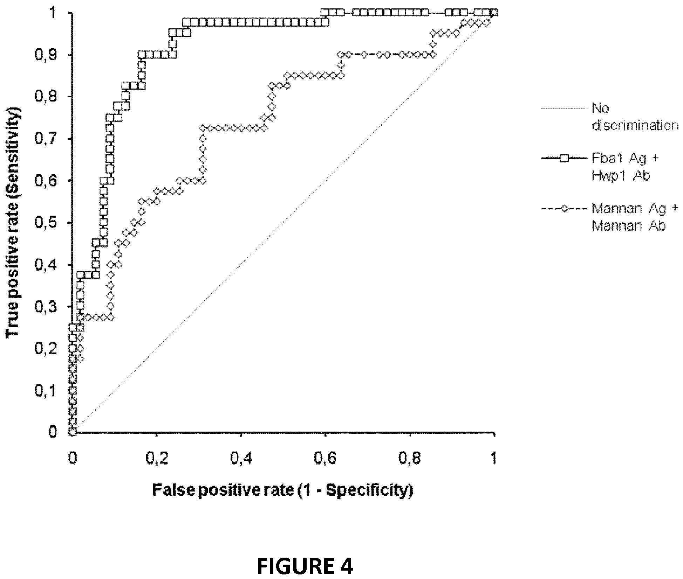

Figure 4. ROC curves showing the diagnostic performances of Candida Fba1 protein and anti-HWP1 antibodies (Hwp1 Ab) association (squares) in comparison with the commercially available tests for the combined detection of Mannan Ag and Mannan Ab (diamonds). The diagonal doted-line corresponds to the random-guess or no-discrimination line.

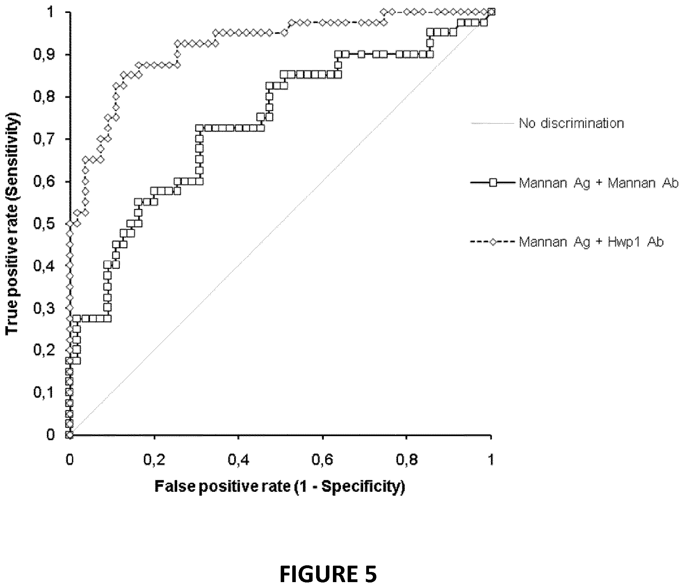

Figure 5. ROC curves showing the diagnostic performance of the Mannan Ag and Hwp1 Ab association (diamonds) in comparison with the commercially available tests for the combined detection of Mannan Ag and Mannan Ab (squares). The diagonal doted-line corresponds to the random-guess or no-discrimination line.

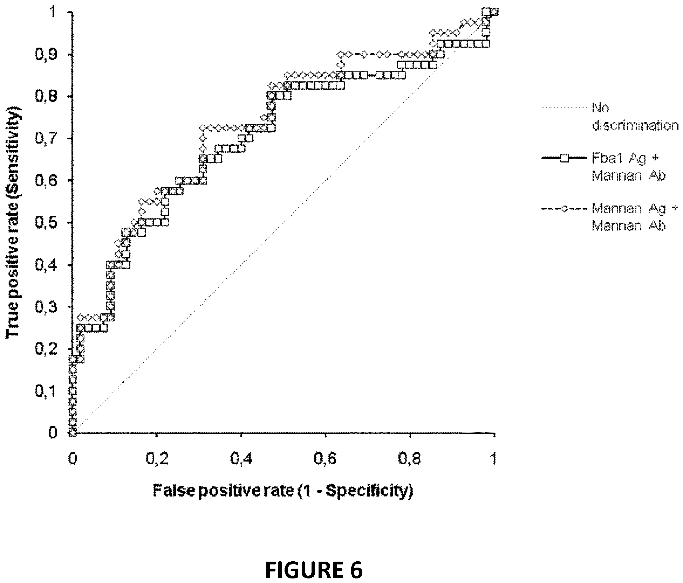

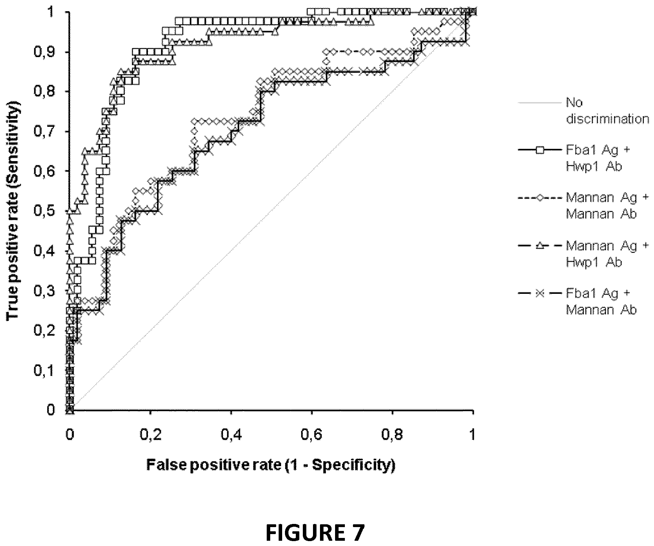

Figure 6*.* ROC curves showing the diagnostic performance of Candida Fba1 protein and Mannan Ab association (squares) in comparison with the commercially available tests for the combined detection of Mannan Ag and Mannan Ab (diamonds). The diagonal doted-line corresponds to the random-guess or no-discrimination line.Figure 7. ROC curves showing the diagnostic performances of of the following biomarkers associations: Candida Fba1 protein and Hwp1 Ab (squares), Mannan Ag and Mannan Ab (diamonds), Mannan Ag and Hwp1 Ab (triangles) and Candida Fba1 protein and Mannan Ab (cross). The diagonal doted-line corresponds to the random-guess or no-discrimination line.

### EXAMPLES

### Example 1: Evaluation of diagnosis potential of Candida Fba1 protein detection

### 1) Materials and methods

### Patients

Between January 2008 and December 2010, 50 serum samples were retrospectively collected in different clinical departments of Lille University Hospital (LUH), from 50 patients (21 females and 29 males [mean age, 61.4 +/- 13.5 years]) with proven *Candida albicans* and non *albicans spp* candidiasis. This group contains 4 different yeast species: *Candida albicans* (29 patients and sera), *Candida glabrata* (9 patients and sera), *Candida parapsilosis* (6 patients and sera) and *Candida tropicalis* (6 patients and sera).

**Table 1. Clinical features of patients with systemic Candida spp infection. Patients were classified according to Candida species and age.**

| **Patients** | **Age (yrs)** | **^{a}Sex** | **Candida species** | **Date of serum sampling in relation to blood culture (days)** |
|---|---|---|---|---|
| 1 | 27 | F | *C albicans* | -1 |
| 2 | 41 | M | *C albicans* | 0 |
| 3 | 41 | M | *C albicans* | 3 |
| 4 | 42 | F | *C albicans* | 1 |
| 5 | 43 | M | *C albicans* | -4 |
| 6 | 44 | F | *C albicans* | -3 |
| 7 | 46 | M | *C albicans* | 0 |
| 8 | 47 | M | *C albicans* | 0 |
| 9 | 50 | F | *C albicans* | 0 |
| 10 | 51 | F | *C albicans* | 0 |
| 11 | 52 | M | *C albicans* | -2 |
| 12 | 55 | M | *C albicans* | 1 |
| 13 | 57 | M | *C albicans* | 0 |
| 14 | 58 | M | *C albicans* | 2 |
| 15 | 59 | M | *C albicans* | 0 |
| 16 | 61 | M | *C albicans* | 1 |
| 17 | 61 | M | *C albicans* | -1 |
| 18 | 66 | F | *C albicans* | 3 |
| 19 | 67 | M | *C albicans* | 0 |
| 20 | 68 | M | *C albicans* | 2 |
| 21 | 70 | M | *C albicans* | -1 |
| 22 | 70 | F | *C glabrata* | 0 |
| 23 | 71 | F | *C albicans* | 3 |
| 24 | 72 | M | *C albicans* | 3 |
| 25 | 77 | M | *C albicans* | 1 |
| 26 | 79 | M | *C albicans* | 0 |
| 27 | 81 | M | *C albicans* | 0 |
| 28 | 81 | F | *C albicans* | 0 |
| 29 | 83 | F | *C albicans* | -1 |
| 30 | 51 | M | *C glabrata* | 2 |
| 31 | 59 | F | *C glabrata* | 0 |
| 32 | 61 | F | *C glabrata* | 2 |
| 33 | 63 | M | *C glabrata* | 0 |
| 34 | 70 | F | *C glabrata* | 0 |
| 35 | 73 | F | *C glabrata* | 2 |
| 36 | 74 | M | *C glabrata* | 4 |
| 37 | 79 | F | *C glabrata* | 0 |
| 38 | 80 | F | *C glabrata* | 0 |
| 39 | 58 | F | *C parapsilosis* | 3 |
| 40 | 60 | F | *C parapsilosis* | 0 |
| 41 | 65 | M | *C parapsilosis* | -2 |
| 42 | 71 | M | *C parapsilosis* | -3 |
| 43 | 75 | M | *C parapsilosis* | -1 |
| 44 | 85 | F | *C parapsilosis* | 3 |
| 45 | 41 | M | *C tropicalis* | 2 |
| 46 | 42 | F | *C tropicalis* | 0 |
| 47 | 55 | F | *C tropicalis* | 0 |
| 48 | 57 | M | *C tropicalis* | -3 |
| 49 | 71 | M | *C tropicalis* | -2 |
| 50 | 72 | M | *C tropicalis* | 0 |

| | | | | |
|---|---|---|---|---|
| ^{a}M, Male; F, Female | | | | |

The following criteria were applied as retrospective selection rules when the laboratory and clinical files were examined: (i) positive blood culture from Candida *species;* (ii) availability of serum samples obtained within a range of 3 weeks before and 1 month after positive cultures, (iii) the presence of risk factors (cancer and chemotherapy, abdominal surgery, AIDS, major health problems requiring hospitalization in intensive care units -ICUs-, and use of broad-spectrum antibiotics, indwelling intravascular catheters, and hyperalimentation); and (iv) the presence of an infectious syndrome (namely, fever) that did not respond to antibacterial therapy but that did respond to antifungal therapy. In order to evaluate performances of biomarkers for the early diagnosis of IC, selection of serum samples was restricted to the period ranging from 5 days before to 5 days after the date of isolation of yeast species from blood culture. After blood sampling blood samples were centrifuged and serum aliquots were stored at -80°C until required.

### Control group.

A group of control sera were included in this study and consisted of 55 serum samples from healthy blood donors.

### EIA detection of Candida Fba 1 antigen in human sera

Specific monoclonal antibodies against Fba1 protein were coated on ELISA plates (NUNC IMMUNO-MODULE 468680) at a concentration of 2 µg/ml, with carbonate solution pH 9.5+/-0.2 filtered 0.22 µm. This preparation was incubated at room temperature overnight.

After coating, the wells were blocked by 100 µl of Bovine Serum Albumin/Saccharose solution pH 7.2+/-0.2 filtered 0.22 µm, emptied and filled with 200 µl of the same solution. Plates were then frozen at -20°C. Protocols for antibodies coating, preparation of diluent solutions, dilution of patient sera, and conjugate solutions were based on preliminary experiments performed with both serum added with a gradual concentration of recombinant Fba1 produced in Escherichia coli and a pool of sera from IC patients known to display high titers of *Candida* antigenemia.

Patient sera were diluted 1/100 in Tris saline buffer and BSA pH 7.6 and were incubated for 1 h at 37°C on coated plate in dry incubator, 3 times washed with 800 µl of Tris saline buffer and Tween 20 (0.1%) and proclin (0.07%), incubated 1 h at 37°C with a secondary anti-Fba1 antibody conjugated to peroxidase (Bio-Rad, Marnes La Coquette, France), washed 3 times as previously described and incubated 30 minutes with 200 µl of tetramethyl benzidine (TMB) and stored at room temperature in dark. The reaction was stopped by the addition of 100 µl of stopping solution containing 2M H₂SO₄ and ODs were measured at 450/620nm. In parallel, all sera were tested at the same time with the home-made EIA tests involving anti-Fba1 monoclonal antibody, Hwp1 recombinant proteins, the Platelia™ Candida Ab Plus and the Platelia™ Candida Ag.

### Detection of mannanemia.

Circulating mannan was detected using the Platelia Candida antigen Plus (Ag) test (Bio-Rad Laboratories, Marnes-La-Coquette, France) as described previously (Sendid et al., J Med Microbiol. 2002 May;51(5):433-42). Briefly, microtiter plates were sensitized in an industrial setting with monoclonal antibody (monoclonal antibody EBCA1 of Platelia Candida antigen Plus (Ag) test). 300 µl of patient sera was denatured with 100 µl of EDTA treatment solution, and the mixture was boiled for 3 min and centrifuged at 10,000 g for 10 min. 50 µl of supernatant, obtained from patient serum and treated as described above, was mixed in a plate well with 50 µl of horseradish peroxidase-conjugated EBCA1. After incubation for 90 min at 37°C, the plates were washed intensively and the reaction was revealed by 30 min of incubation in darkness with 200 µl of tetramethylbenzidine solution. The optical density was read at a λ of 450/620 nm on a PR2100 reader (Sanofi Pasteur Diagnostics). Reactions were performed in duplicate. Each experiment included a calibration curve for a pool of normal human sera supplemented with concentrations of mannan of 0.1 to 27 ng/ml.

### Statistical analysis

Statistical analysis was performed with the Analyse-it soft version 2.25 Excel 12+. Discrimination between IC and CTRL was performed with Mann-Whitney test while comparisons of diagnosis potentials of all biomarkers were evaluated with Wilcoxon test. A p-value below 0.05 was considered as statistically significant. All data distributions are illustrated as medians and boxplots for each biomarker.

The marker diagnostic performance could be characterised by sensitivity, which represents its ability to detect the IC population, and specificity which represents its ability to detect the control population.

The results of the evaluation of a diagnostic test can be summarised in a 2x2 contingency table comparing these two well-defined populations. By fixing a cut-off the two populations could be classified into categories according to the results of the test, categorised as either positive or negative. Given a particular marker, we can identify a number of subjects with a positive test result among the "cases" population (*the "True Positive": TP*) and *b* subjects with a positive test result among the "controls" population (*the "True Negative": TN*)*.* In the same fashion, *c* subjects with a negative test result among the cases *(the "False Positive": FP)* and *d* subjects with a negative test result among the controls *(the "False Negative": FN)* are observed. Sensitivity is defined as TP/(TP+FN); which is herein referred to as the "true positive rate". Specificity is defined as TN/(TN+FP); which is herein referred to as the "true negative rate".

The accuracy of each marker and its discriminatory power was evaluated using a Receiving Operating Characteristics (ROC) analysis. ROC curves are the graphical visualization of the reciprocal relation between the sensitivity (Se) and the specificity (Sp) of a test for various values.

### 2) Results

### Standardization of tests.

For each experiment, 3 serum controls were used. Negative control collected from healthy subject and 2 positive sera consisted of 1 pool of sera with known reactivity against *Candida albicans* mannan and one serum collected from patient belonging to IC group selected from previous series of experiments. All these controls allowed us to reduce inter-experiments variations.

A study of diagnosis potential of Candida Fba1 antigen was performed by comparison of medians. An interesting potential was obtained with similar performances that current antigen diagnosis test Platelia™ Candida antigen plus but practicability is better (volume of biological samples, no treatment).

### Comparison of serological reactivity against a panel of antigens within IC and control groups

Using IC and control groups of patients, boxplots were performed for Candida Fba1 and mannan antigens (Figures 1-2). Candida Fba1 and mannan antigens have shown a significant discrimination between IC and controls (p<0.0001) and when these 2 biomarkers were compared, significant difference was obtained (p<0.0001; figure 1).

### Comparison of sensitivity and specificity of mannanemia and RP-Ab combined analysis.

Retrospective analysis of the cohort allowed sensitivity, specificity, PPV, NPV and accurancy of 38.0%, 100%, 100%, 70.5% and 77.5%, respectively for mannanemia. In comparison, for Candida Fba1 antigen calculated sensitivity, specificity, PPV, NPV and accurancy were of 42%, 100%, 100%, 72.4% and 79.7%, respectively.

The ROC curves obtained from these two markers alone showed similar diagnosis performances as reveled by AUCs: 0.70 and 0.71 Candida Fba1 and mannan antigenemia (Figure 3, Table 2).

**Table 2. Diagnosis potential (mROC approach) of Candida Fba1 and mannan antigen for IC diagnosis.**

| | **AUC** | **Cut-off** | **Sp (%)** | **Se (%)** | **PPV (%)** | **NPV (%)** | **Accuracy (%)** | **CI 95%** |
|---|---|---|---|---|---|---|---|---|
| **Mannan** | 0.71 | 0.1 | 100.0 | 38.0 | 100.0 | 70.5 | 77.5 | [0.64; 0.78] |
| **Fba1** | 0.77 | 0.29 | 100.0 | 42.0 | 100.0 | 79.7 | 79.5 | [0.68; 0.86] |

AUC: area under the curve; Se: sensibility; Sp: specificity; PPV: positive predictive value (measures the proportion of subjects with positive test results who are correctly diagnosed); NPV: negative predictive value (measures the proportion of subjects with negative test results who are correctly diagnosed); CI 95%: 95% confidence interval. The cut-off value was set in order to specificity to 80%.

### Example 2. Analysis of discriminatory potential of biomarkers combination in

### compared with the mannanemia and anti-mannan antibody association

### 1) Materials and methods

### Patients

40 serum samples were retrospectively collected in different clinical departments of Lille University Hospital (LUH), from 40 patients (16 females and 24 males [mean age, 61.4 +/- 14.7 years]) with proven *Candida albicans* and non *albicans spp* candidiasis. This group contains 4 different yeast species: *Candida albicans* (24 patients and sera), *Candida glabrata* (8 patients and sera), *Candida parapsilosis* (4 patients and sera) and *Candida tropicalis* (4 patients and sera).

To improve the diagnostic potential of Fba1 antigen, combinations were explored with three other biomarkers selected from the group consisted of specific antibodies directed against hyphal wall protein 1 (Hwp1), specific antibodies directed against mannan and the circulating mannan antigen from *Candida spp.*

### EIA detection of anti-C. albicans recombinant proteins antibodies in human sera

Recombinant Hwp1 proteins were coated on ELISA plates (NUNC IMMUNO-MODULE 468680) at a concentration of 2 µg/ml of Hwp1 produced in *Escherichia coli,* with carbonate solution pH 9.5+/-0.2 filtered 0.22 µm for all antigens. These preparations were incubated at room temperature overnight.

After coating, the wells were blocked by 100 µl of Bovine Serum Albumin/Saccharose solution pH 7.2+/-0.2 filtered 0.22 µm, emptied and filled with 200 µl of the same solution. Plates were then frozen at -20°C. Protocols for antigens coating, preparation of diluent solutions, dilution of patient sera, and conjugate solutions were based on preliminary experiments performed with a pool of sera from IC patients known to display high titers of anti-*Candida* antibodies.

Patient sera were diluted 1/100 in Tris saline buffer and BSA pH 7.6 and were incubated for 1 h at 37°C on coated plate in dry incubator, 3 times washed with 800 µl of Tris saline buffer and Tween 20 (0.1%) and proclin (0.07%), incubated 1 h at 37°C with a secondary anti-total immunoglobulin antibody conjugated to peroxidase (Bio-Rad, Marnes La Coquette, France), washed 3 times as previously described and incubated 30 minutes with 200 µl of tetramethyl benzidine (TMB) and stored at room temperature in dark. The reaction was stopped by the addition of 100 µl of stopping solution containing 2M H₂SO₄ and ODs were measured at 450/620nm. In parallel, all sera were tested at the same time with the home-made EIA tests involving recombinant proteins, the Platelia™ Candida Ab Plus and the Platelia™ Candida Ag.

### EIA detection of anti-C. albicans mannan antibodies in human sera.

For comparison purposes, antibodies to *Candida albicans* mannan were detected using the Platelia Candida antibody (Ab) Plus test (Bio-Rad Laboratories, Marnes La-Coquette, France) according to manufacturer's instructions. Briefly, Enzyme ImmunoAssay (EIA) was performed with BEP III automate (Behring Laboratories, Paris, France). For individual sera, 100 µl of serum diluted 1/400 was applied to each well, and the plate was incubated for 1 h at 37°C. After washing, 100 µl of horseradish peroxidase-conjugated anti-human immunoglobulins was then added, and the plates were incubated for 1 h at 37°C. After intensive washing, the reaction was revealed by 30 min of incubation in darkness with 200 µl of tetramethylbenzidine solution. The absorbance at a λ of 450/620 nm was measured. The results were reported in arbitrary units (AU) in relation to the results on the standard curve.

### 2) Results

With a specificity arbitrarily fixed at 80.0%, combination of Fba1/anti-Hwp1 antibody association was shown sensitivity, PPV, NPV and accuracy of 92.5%, 77.1%, 93.6%, 80.2% (Figure 4), respectively while mannanemia/anti-mannan antibodies (current commercialized diagnosis tests) lead to 77.5%, 73.8%, 83.0% and 74.3% (Figure 4 to 7), respectively mannanemia/anti-Hwp1 lead to 87.5%, 76.1%, 89.8% and 78.2% (Figure 5), respectively and eventually Fba1/anti-mannan antibody lead to 80.0%, 74.4%, 84.6% and 75.2% (Figure 6), respectively for IC patients *versus* blood donors (Table 3). All these results were compiled in a comparison ROC curves represented in figure 7.

**Table 3. Diagnosis potential (mROC approach) of association of biomarkers for IC diagnosis.**

| | **AUC** | **Sp (%)** | **Se (%)** | **PPV (%)** | **NPV (%)** | **Accuracy (%)** | **CI 95%** |
|---|---|---|---|---|---|---|---|
| **Mannan Ag + Mannan Ab** | 0.73 | 80.0 | 77.5 | 73.8 | 83.0 | 74.3 | [0.63; 0.84] |
| **Mannan Ag + Hwp1 Ab** | 0.92 | 80.0 | 87.5 | 76.1 | 89.8 | 78.2 | [0.86; 0.97] |
| **Fba1 + Mannan Ab** | 0.70 | 80.0 | 80.0 | 74.4 | 84.6 | 75.2 | [0.59; 0.82] |
| **Fba1 + Hwp1** | 0.91 | 80.0 | 92.5 | 77.1 | 93.6 | 80.2 | [0.86; 0.97] |

In the end, the combined detection of circulating Fba1 protein and anti-Hwp1 antibodies make it possible to increase by 20 % (by adding increases in sensitivity and specificity) the diagnostic performance compared to the combination of the commercial Platelia™ tests, and by 5% compared to the combination of detection of mannan antigen and anti-Hwp1 antibodies.

Compared analysis of the detection of mannan antigen, anti-mannan antibodies, Fba1 protein or anti-Hwp1 antibodies within a series of 40 sera of patients with invasive candidiasis shows a lack of correlation in mannan or Fba1 antigen detection leading to different patterns of detection (Table 4). Interestingly, Fba1 and anti-Hwp1 antibody determinations are complementary which makes it possible, by combining both Fba1 and anti-Hwp1 antibody determinations, to achieve the best detection of positive samples within the IC sera.

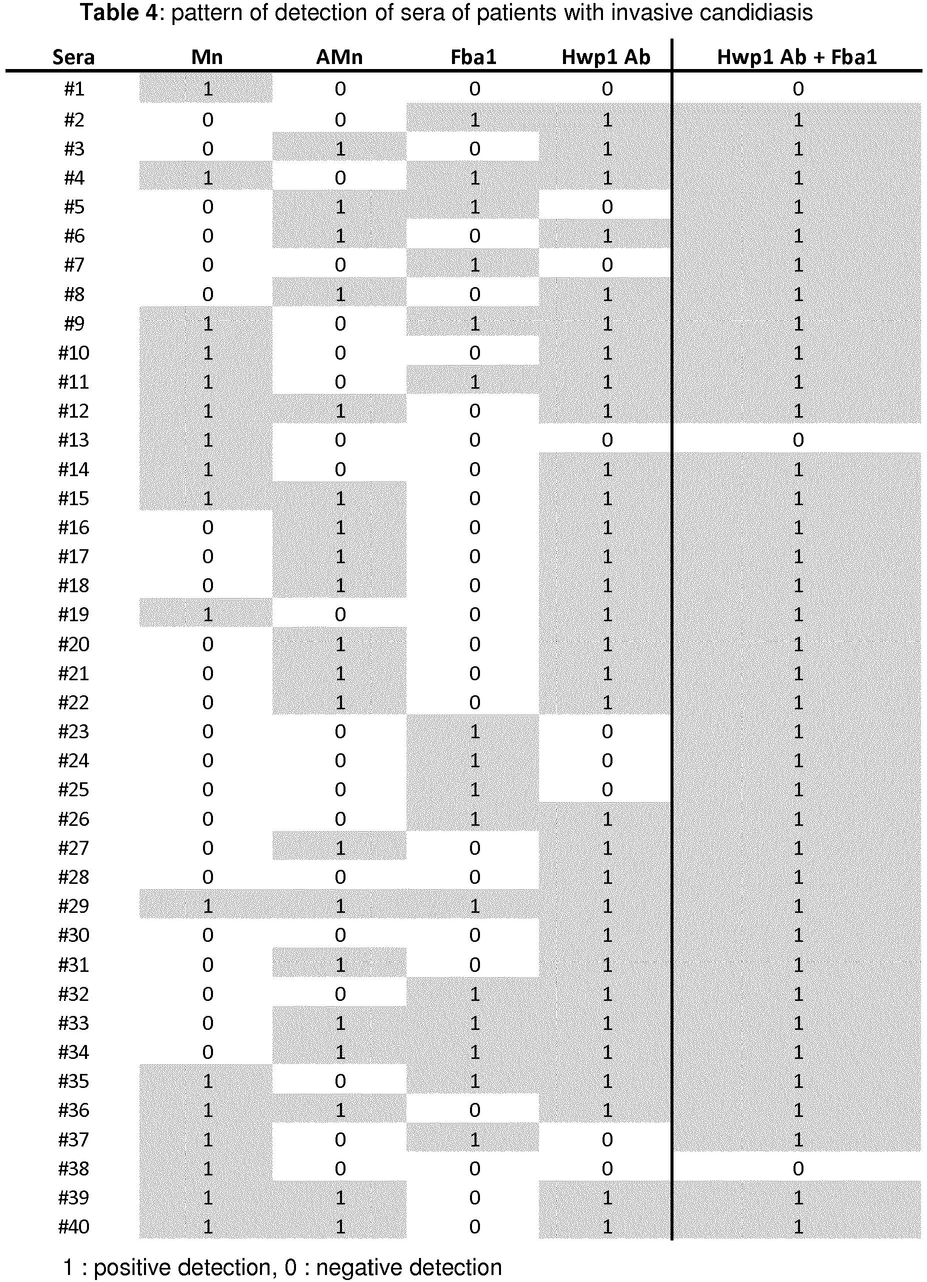

Altogether, these results indicate that anti-Hwp1/Fba1 association can advantageously substitute anti-mannan antibodies/mannanemia for the prediction of IC and even anti-Hwp1/mannanemia and Fba1/anti-mannan antibody combination.

Such a result was unexpected because whereas detection of mannan and anti-mannan antibodies is complementary in patients with invasive candidiasis, most probably due to the implication of anti-mannan antibodies in the clearance of soluble mannan, such a complementarity could not be expected for Fba1 and anti-Hwpl, based on a mechanism of antigen clearance. Therefore, to our knowledge, it's the first demonstration of specific detection of circulating Fba1 protein detection for the diagnosis of candidemia. Moreover, it was entirely surprising that Fba1 antigen and anti-Hwp1 detections were also complementary and could be improved invasive candidiasis diagnosis.

## Claims

1. An *in vitro* method for diagnosing invasive candidiasis (IC) in a subject, said method comprising the steps of:
a) detecting the presence of a Candida fructose bisphosphate aldolase (Fba1) protein in a first blood, plasma or serum sample of the subject,
b) determining that the subject has IC based upon the presence of said Candida Fba1 protein in said blood, plasma or serum sample of the subject.

2. The method according to claim 1, which further comprises the step of detecting the presence of antibodies directed against Candida hyphal wall protein (Hwp1) in a second blood or plasma sample of said subject, and determining that the subject has IC based upon the presence of antibodies directed against Candida Hwp1 protein in said second blood or plasma sample of said subject.

3. The method according to claim 1 or 2, wherein Candida Fba1 protein is detected by immunoassay.

4. The method according to any one of claims 1 to 3, wherein Candida Fba1 protein is detected by immunoassay using a monoclonal antibody, or a fragment thereof, directed against Candida Fba1 protein.

5. The method according to any one of claims 2 to 4, wherein antibodies directed against Candida Hwp1 protein are detected by immunoassay.

6. The method according to any one of claims 2 to 5, wherein antibodies directed against Candida Hwp1 protein are detected by immunoassay using a polypeptide comprising, or consisting of :
a) SEQ ID NO:4 or a naturally existing variant or isoform thereof;
b) an epitopic fragment of a polypeptide defined in a), in particular a fragment consisting of amino acids at positions 41 to 200, or 27 to 203 of SEQ ID NO:4, or a variant thereof.

7. The method according to any one of claims 2 to 6, wherein said first and second samples are a same sample or aliquots from a same sample.

8. A method for determining a treatment regimen in a subject, said method comprising:
a) diagnosing if the subject has invasive candidiasis by carrying out the method defined in any one of claims 1 to 7, and
b) if the subject is diagnosed as having invasive candidiasis, determining an antifungal treatment for said subject.

9. A kit for diagnosing invasive candidiasis comprising:
a) means for determining the presence or level of a Candida Fba1 protein, and
b) means for determining the presence or level of antibodies directed against Candida Hwp1 protein.

## Patentansprüche

1. Ein *in vitro* Verfahren zum Diagnostizieren von invasiver Candidiasis (IC) in einem Individuum, wobei das Verfahren die Schritte umfasst:
a) Nachweisen des Vorliegens eines Candida-Fruktose-Bisphosphat-Aldolase-(Fba1)-Proteins in einer ersten Blut-, Plasma- oder Serumprobe des Individuums,
b) Bestimmen, dass das Individuum IC aufweist, basierend auf dem Vorliegen des Candida-Fba1-Proteins in der Blut-, Plasma- oder Serumprobe des Individuums.

2. Das Verfahren gemäß Anspruch 1, das ferner den Schritt des Nachweisens des Vorliegens von, gegen ein Candida Hyphenwandprotein (Hwp1) gerichtete Antikörper in einer zweiten Blut- oder Plasmaprobe des Individuums umfasst und Bestimmen, dass das Individuum IC aufweist, basierend auf dem Vorliegen der gegen das Candida-Hwp1-Protein gerichteten Antikörper in der zweiten Blut- oder Plasmaprobe des Individuums.

3. Das Verfahren gemäß Anspruch 1 oder 2, wobei das Candida-Fba1-Protein durch einen Immunassay nachgewiesen wird.

4. Das Verfahren gemäß einem der Ansprüche 1 bis 3, wobei das Candida-Fba1-Protein durch den Immunassay, durch Verwenden eines, gegen das Candida-Fba1-Protein gerichteten monoklonalen Antikörpers oder eines Fragments davon, nachgewiesen wird.

5. Das Verfahren gemäß einem der Ansprüche 2 bis 4, wobei Antikörper, die gegen Candida-Hwp1 Protein gerichtet sind durch den Immunassay nachgewiesen werden.

6. Das Verfahren gemäß einem der Ansprüche 2 bis 5, wobei Antikörper, die gegen Candida-Hwp1 Protein gerichtet sind durch den Immunassay nachgewiesen werden, durch Verwenden eines Polypeptids umfassend oder bestehend aus:
a) SEQ ID NO:4 oder einer natürlich vorkommenden Variante oder Isoform davon;
b) einem Epitopfragment eines Polypeptids definiert in a) insbesondere einem Fragment bestehend aus Aminosäuren an den Positionen 41 bis 200 oder 27 bis 203 der SEQ ID NO:4 oder einer Variante davon.

7. Das Verfahren gemäß einem der Ansprüche 2 bis 6, wobei die ersten und zweiten Proben eine gleiche Probe oder Aliquots einer gleichen Probe sind.

8. Ein Verfahren zum Bestimmen eines Behandlungsschemas in einem Individuum, wobei das Verfahren umfasst:
a) Diagnostizieren, ob das Individuum eine invasive Candidiasis aufweist, durch Durchführen des Verfahrens wie in einem der Ansprüche 1 bis 7 definiert, und
b) wenn das Individuum als die invasive Candidiasis aufweisend diagnostiziert wurde, Bestimmen einer antimykotischen Behandlung des Individuums.

9. Ein Kit zum Diagnostizieren der invasiven Candidiasis, umfassend:
a) Mittel zum Bestimmen des Vorliegens oder der Menge eines Candida-Fba1-Proteins und
b) Mittel zum Bestimmen des Vorliegens oder der Menge von Antikörpern, die gegen das Candida-Hwp1-Protein gerichtet sind.

## Revendications

1. Procédé *in vitro* pour diagnostiquer une candidose invasive (CI) chez un sujet, ledit procédé comprenant les étapes consistant à :
a) détecter la présence d'une protéine fructose-bisphosphate aldolase (Fba1) de Candida dans un premier échantillon de sang, de plasma ou de sérum du sujet,
b) déterminer que le sujet présente une CI en se basant sur la présence de ladite protéine Fba1 de Candida dans ledit échantillon de sang, de plasma ou de sérum du sujet.

2. Procédé selon la revendication 1, qui comprend en outre l'étape consistant à détecter la présence d'anticorps dirigés contre une protéine de paroi hyphale (Hwp1) de Candida dans un second échantillon de sang ou de plasma dudit sujet, et à déterminer que le sujet présente une CI en se basant sur la présence des anticorps dirigés contre la protéine Hwp1 de Candida dans ledit second échantillon de sang ou de plasma dudit sujet.

3. Procédé selon la revendication 1 ou 2, dans lequel la protéine Fba1 de Candida est détectée par un dosage immunologique.

4. Procédé selon l'une quelconque des revendications 1 à 3, dans lequel la protéine Fba1 de Candida est détectée par un dosage immunologique en utilisant un anticorps monoclonal, ou un fragment de celui-ci, dirigé contre la protéine Fba1 de Candida.

5. Procédé selon l'une quelconque des revendications 2 à 4, dans lequel les anticorps dirigés contre la protéine Hwp1 de Candida sont détectés par un dosage immunologique.

6. Procédé selon l'une quelconque des revendications 2 à 5, dans lequel les anticorps dirigés contre la protéine Hwp1 de Candida sont détectés par un dosage immunologique en utilisant un polypeptide comprenant, ou consistant en :
a) SEQ ID NO: 4 ou un variant ou une isoforme de celle-ci existant à l'état naturel ;
b) un fragment épitopique d'un polypeptide défini en a), en particulier un fragment consistant en les acides aminés aux positions 41 à 200, ou 27 à 203 de SEQ ID NO: 4, ou un variant de celui-ci.

7. Procédé selon l'une quelconque des revendications 2 à 6, dans lequel lesdits premier et second échantillons sont un même échantillon ou des aliquotes d'un même échantillon.

8. Procédé pour déterminer un régime thérapeutique chez un sujet, ledit procédé comprenant :
a) le fait de diagnostiquer si le sujet présente une candidose invasive en réalisant le procédé défini dans l'une quelconque des revendications 1 à 7, et
b) si le sujet est diagnostiqué comme présentant une candidose invasive, déterminer un traitement antifongique pour ledit sujet.

9. Kit pour diagnostiquer une candidose invasive comprenant :
a) un moyen pour déterminer la présence ou le taux d'une protéine Fba1 de Candida, et
b) un moyen pour déterminer la présence ou le taux d'anticorps dirigés contre une protéine Hwp1 de Candida.
